(19) Europäisches Patentamt

European Patent Office

Office européen des brevets

(11) Veröffentlichungsnummer: **0 295 549 B1**

(12) # EUROPÄISCHE PATENTSCHRIFT

(45) Veröffentlichungstag der Patentschrift: **18.08.93**

(21) Anmeldenummer: **88109085.6**

(22) Anmeldetag: **08.06.88**

Die Akte enthält technische Angaben, die nach dem Eingang der Anmeldung eingereicht wurden und die nicht in dieser Patentschrift enthalten sind.

(51) Int. Cl.⁵: **C07C 67/333**, C07C 67/38, C07C 69/533, C07C 69/716, C07C 69/67

(54) **Verfahren zur Herstellung von 5-Formylvaleriansäureestern.**

(30) Priorität: **15.06.87 DE 3719933**

(43) Veröffentlichungstag der Anmeldung:
**21.12.88 Patentblatt 88/51**

(45) Bekanntmachung des Hinweises auf die Patenterteilung:
**18.08.93 Patentblatt 93/33**

(84) Benannte Vertragsstaaten:
**BE CH DE ES FR GB IT LI NL**

(56) Entgegenhaltungen:
**EP-A- 0 031 100**
**US-A- 4 035 408**
**US-A- 4 517 400**

(73) Patentinhaber: **BASF Aktiengesellschaft**
**Carl-Bosch-Strasse 38**
**D-67063 Ludwigshafen(DE)**

(72) Erfinder: **Bertleff, Werner, Dr.**
**Neuzenlache 3**
**D-6806 Viernheim(DE)**
Erfinder: **Fischer, Rolf, Dr.**
**Bergstrasse 98**
**D-6900 Heidelberg(DE)**
Erfinder: **Kummer, Rudolf, Dr.**
**Kreuzstrasse 6**
**D-6710 Frankenthal(DE)**
Erfinder: **Merger, Franz, Dr.**
**Max-Slevogt-Strasse 25**
**D-6710 Frankenthal(DE)**
Erfinder: **Schneider, Heinz-Walter, Dr.**
**Brüsseler Ring 43**
**D-6700 Ludwigshafen(DE)**

## Beschreibung

Aus der europäischen Patentschrift 31 100 ist bekannt, daß man 5-Formylvaleriansäureester durch Hydroformylieren von Pentensäureestern erhält. Hierbei fällt ein Gemisch aus Formylvaleriansäureestern an, das den gewünschten 5-Formylvaleriansäureestern zu 70 % enthält. Es wurde auch schon versucht, 3-Pentensäureester zunächst zu 4-Pentensäureester zu isomerisieren und diesen dann zu hydroformylieren, um den Anteil an geradkettigen Formylvaleriansäureestern zu erhöhen wie aus der europäischen Patentschrift 125 567 zu entnehmen ist. Abgesehen davon, daß ein zusätzlicher Isomerisierungsschritt aufwendig ist, erhält man immer noch beachtliche Anteile an nichtverwertbaren verzweigten Formylvaleriansäureestern.

Es war deshalb die technische Aufgabe gestellt ein Verfahren zur Herstellung von 5-Formylvaleriansäureestern ausgehend von Pentensäureestern zur Verfügung zu stellen, bei dem Pentensäureester möglichst vollständig in 5-Formylvaleriansäureester übergeführt werden, eine zusätzliche Isomerisierung von Pentensäureestern zu 4-Pentensäureester nicht erforderlich ist und schließlich der Anfall von nichtverwertbaren verzweigten Formylvaleriansäureestern und weiteren Nebenprodukten weitgehend vermieden wird.

Diese Aufgabe wird gelöst in einem Verfahren zur Herstellung von 5-Formylvaleriansäureestern welches folgende Schritte umfaßt

a) Hydroformylierung von Pentensäureestern durch Umsetzen mit Kohlenmonoxid und Wasserstoff bei einer Temperatur von 60 bis 160°C und unter erhöhtem Druck in Gegenwart von Carbonylkomplexen von Metallen der 8. Gruppe des periodischen Systems unter Bildung eines Gemisches von 5,4- und 3-Formylvaleriansäureestern

b) Abtrennen von 5-Formylvaleriansäureestern aus dem so erhaltenen Gemisch von 5,4- und 3-Formylvaleriansäureestern unter Verbleib eines im wesentlichen aus 4- und 3-Formylvaleriansäureestern bestehenden Gemisches

c) Spalten des im wesentlichen aus 4- und 3-Formylvaleriansäureester bestehenden Gemisches in Pentensäureester bei einer Temperatur von 50 bis 400°C in Gegenwart von Katalysatoren, die ein oder mehrere Metalle der 8. Nebengruppe des Periodischen Systems enthalten, und

d) Zurückführen der Pentensäureester in Stufe a zur Hydroformylierung

Das neue Verfahren hat den Vorteil, daß man aus Pentensäureestern ohne vorherige Isomerisierung zur 4-Pentensäureestern 5-Formylvaleriansäureester erhält unter Minimierung des Anfalls von Nebenprodukten.

Dies war nicht zu erwarten, da entsprechend der EP-Anmeldung 81090 Formylvaleriansäureester beim Erhitzen auf eine Temperatur von 200 bis 350°C in Gegenwart von Katalysatoren Dihydropyrone ergeben.

In der Stufe a werden Pentensäureester hydroformyliert. Geeignete Pentensäureester leiten sich von Alkanolen mit 1 bis 12 Kohlenstoffatomen und Cycloalkanolen mit 5 bis 8 Kohlenstoffatomen ab. Besonders bevorzugt sind Pentensäure $C_1$ bis $C_6$-Alkylester, insbesondere Pentensäure $C_1$ bis $C_4$-Alkylester, besonders die Methylester. Geeignet sind 4-Pentensäureester, 3-Pentensäureester und 2-Pentensäureester einzeln oder Gemische derselben. Beispielsweise seien genannt Methyl-, Ethyl-, Propyl-, Isopropyl-, Butyl-, tert.-Butyl-, Hexyl-, Nonyl-, Dodecyl-, Cyclopentyl-, Cyclohexylester, von jeweils 2-Pentensäure, 3-Pentensäure oder 4-Pentensäure.

Die Hydroformylierung der Pentensäureester erfolgt in an sich bekannter Weise z.B. nach EP 31 100. Die Umsetzung wird bei einer Temperatur von 60 bis 160°C, insbesondere von 80 bis 110°C durchgeführt. Hierbei hält man erhöhten Druck vorteilhaft von 50 bis 300 bar insbesondere von 90 bis 150 bar ein. Die Hydroformylierung der Pentensäureester erfolgt durch Umsetzen mit Kohlenmonoxid und Wasserstoff. In der Regel enthält das Gasgemisch Kohlenmonoxid und Wasserstoff im Molverhältnis 1:0,5 bis 1:10, insbesondere 1:1 bis 1:2.

Die Hydroformylierung wird in Gegenwart von Carbonylkomplexen von Metallen der 8. Nebengruppe des periodischen Systems durchgeführt. Besonders geeignet sind Kobalt- und Rhodiumcarbonylkomplexe. Besonders bewährt haben sich Kobaltcarbonylkomplexe. Vorteilhaft wendet man 0,01 bis 1 Mol.% vorzugsweise 0,05 bis 0,3 Mol.%, insbesondere 0,08 bis 0,25 Mol.% Cobaltcarbonylkomplexe berechnet als Cobalt, bezogen auf eingesetzte Pentensäureester an. Vorteilhaft hält man beim Hydroformylieren einen Umsatz der eingesetzten Pentensäureester von 10 bis 50 %, vorzugsweise 20 bis 40 % ein. Hierdurch wird die Bildung von Nebenprodukten durch Hydrieren und Aldolisieren vermindert. Unter diesen Bedingungen ist es möglich, auf die Mitverwendung von Lösungsmitteln zu verzichten und Cobaltkatalysatoren die einen Gehalt von bis zu 20 Mol je Mol Cobalt an tert.- Stickstoffbasen enthalten zu verwenden ohne daß die Hydroformylierung negativ beeinflußt wird. Solche Katalysatoren fallen z. B. bei der Hydroesterifizierung von Butadien zu Pentensäureestern an wie sie in der europäischen Patentschrift 31 100 beschrieben wird.

Es ist jedoch auch möglich, die Hydroformylierung in Gegenwart von unter Reaktionsbedingungen inerten Lösungsmitteln durchzuführen. Geeignete Lösungsmittel sind beispielsweise Ether wie Tetrahydrofuran, ferner Toluol oder Carbonsäureester wie Valeriansäureester, Buttersäureester oder Essigsäureester.

Das Hydroformylierungsgemisch wird nach dem Entspannen nach bekannten Methoden aufgearbeitet. Eine geeignete Methode ist beispielsweise beschrieben in der europäischen Patentschrift 31 100. Hierbei wird der Hydroformylierungsaustrag nach dem Entspannen mit Oxidationsmitteln wie Wasserstoffperoxid oder molekularen Sauerstoff enthaltenden Gasen insbesondere Luft vorteilhaft in einer Menge von 2 bis 10 Oxidationsäquivalenten je Mol Cobalt-Verbindung in Gegenwart einer wäßrigen sauren Lösung z. B. wäßriger Ameisensäure, Essigsäure oder höheren Carbonsäuren wie Buttersäure, Valeriansäure, 2-Ethylhexansäure bei einer Temperatur z.B. von 80 bis 160°C insbesondere 100 bis 130°C behandelt. Je nach dem Grad der Durchmischung ist die Abtrennung des Kobaltkatalysators bereits nach wenigen Sekunden oder Bruchteilen von Sekunden beendet. Die Cobalt enthaltende wäßrige Phase wird zweckmäßig durch Dekantieren abgetrennt. Man erhält als organische Phase ein Gemisch aus 5-, 4- und 3-Formylvaleriansäureestern, das als Nebenprodukte noch Hochsieder und Valeriansäureester sowie gegebenenfalls nicht umgesetzte Pentensäureester enthält.

Diese organische Phase wird durch fraktionierende Destillation so aufgetrennt, daß man gegebenenfalls verwendetes Lösungsmittel und nicht umgesetzte Pentensäureester einzeln oder als Gemisch abtrennt und zweckmäßig in die Hydroformylierungsstufe zurückführt. Aus dem verbleibenden 5-, 4- und 3-Formylvaleriansäureester enthaltendem Gemisch wird 5-Formylvaleriansäureester durch Destillation abgetrennt unter Verbleib eines Gemisches im wesentlichen aus 4- und 3-Formylvaleriansäureestern (Stufe b).

In der nachfolgenden Stufe c wird das so erhaltene Gemisch im wesentlichen aus 4- und 3-Formylvaleriansäureestern unter Bildung von Pentensäureestern bei einer Temperatur von 50 bis 400°C in Gegenwart von Katalysatoren gespalten.

Für die erfindungsgemäße Spaltung können zwar die reinen 4- und 3-Formylvaleriansäureester verwendet werden. Im allgemeinen verwendet man jedoch zweckmäßig die anfallenden Gemische, die in Abhängigkeit vom Wirkungsgrad der Destillation auch noch 5-Formylvaleriansäureester enthalten können. Ein typisches Gemisch enthält beispielsweise 60 bis 75 Gew.% 4-Formylvaleriansäureester 25 bis 35 Gew.% 3-Formylvaleriansäureester sowie bis zu 5 Gew.% 5-Formylvaleriansäureester. Als Spaltprodukte erhält man 4-, 3- und 2-Pentensäureester, vorwiegend 3-Pentensäureester, neben Kohlenmonoxid und Wasserstoff.

Man verwendet in Stufe c Katalysatoren, die ein oder mehrere Elemente der 8. Nebengruppe des periodischen Systems enthalten und vorteilhaft zusätzlich ein oder mehrere Elemente der 1. bis 7. Nebengruppe und/oder Elemente des Seltenen Erden enthalten können.

Vorteilhaft verwendet man als homogene Katalysatoren Komplexverbindungen von Edelmetallen der 8. Nebengruppe insbesondere Ruthenium oder Rhodium. Insbesondere sind Halogene, wie Chlor oder Brom, und Phosphine oder Phosphite enthaltende Ruthenium oder Rhodiumkomplexe geeignet, die zusätzlich als Liganden Kohlenmonoxid enthalten können. Besonders bevorzugt werden als Modifizierungsmittel tertiäre organische Phosphine verwendet. Bevorzugte Substituenten solcher Phosphine sind Alkylreste mit bis zu 18 Kohlenstoffatomen, Cycloalkylreste mit 5 bis 12 Kohlenstoffatomen, Aralkylreste mit 7 bis 10 Kohlenstoffatomen oder Arylreste mit 6 bis 10 Kohlenstoffatome, insbesondere Phenylreste. Die Reste können gleich oder verschieden sein. Beispiele für geeignete Komplexverbindungen sind $RhCl[P(C_6H_5)_3]$, $Ru_2Cl_3[(P(C_6H_5)(C_2H_5)_2)_6]Cl$, $RhBr(CO)[P(C_6H_5)_3]_2$, $HRuCl(CO)[P(C_6H_5)_3]_3$, $RhCl(CO)[P(C_6H_5)_3]_2$.

Bevorzugt werden Trägerkatalysatoren verwendet, die mindestens eines der Elemente der 8. Nebengruppe des Periodischen Systems wie Palladium, Platin, Ruthenium, Rhodium, Osmium, Iridium, Eisen, Kobalt oder Nickel insbesondere Edelmetalle enthalten. Vorteilhaft sind auch Trägerkatalysatoren, die mindestens 2 Edelmetalle, der 8. Nebengruppe wie Ruthenium, Rhodium, Palladium, Platin, Iridium oder Osmium, enthalten. Andere bevorzugte Katalysatoren enthalten mindestens ein Metall der vorgenannten Edelmetalle der 8. Nebengruppe des Periodischen Systems und zusätzlich mindestens ein Metall, ausgewählt aus der Gruppe Eisen, Kobalt und Nickel.

Die Trägerkatalysatoren haben vorteilhaft ein Gehalt an aktiven Metallen der 8. Nebengruppe des Periodischen Systems von 0,01 bis 10, vorzugsweise 0,05 bis 5, insbesondere 0,05 bis 1 Gew.%, bezogen auf die Summe aus Träger und katalystisch aktiven Metallen berechnet als Metall. Als Träger werden vorteilhaft Aluminiumoxid, Siliciumdioxid, Titandioxid, Zinkoxid, Lauthanoxid, Bariumsulfat Zirkondioxid oder Gemische dieser Oxide sowie Aluminiumsilikate verwendet.

Besonders vorteilhaft enthalten die genannten Trägerkatalysatoren noch mindestens ein Element der 1. bis 7. Nebengruppe und/oder Elemente der Seltenen Erden, z. B. Zink, Kupfer, Silber, Lauthan, Titan, Vanadium, Chrom, Molybdän, Wolfram, Mangan, Rhenium, Cer, Neodym oder Praseodym vorteilhaft in einer Menge von 0,05 bis 2 Gew.%, bezogen auf das Gesamtgewicht des Katalysators (Träger und katalytisch aktive Metalle) berechnet als Metalle.

Bewährt haben sich z. B. Tränkkatalysatoren bei denen die katalytisch wirksamen Metalle an der Oberfläche des Trägers angereichert sind. Derartige Katalysatoren werden in an sich bekannter Weise durch Tränken vorgeformter Träger wie Pellets, Kugeln oder Stränge mit einer wäßrigen Lösung der Metallsalze z. B. der Nitrate erhalten, die dann getrocknet, calciniert und direkt oder gegebenenfalls nach Reduktion mit Wasserstoff oder anderen Reduktionsmitteln eingesetzt werden können.

Die in Stufe c verwendeten Katalysatoren zeigen über einen längeren Zeitraum hohe Aktivität. Verbrauchte Katalysatoren lassen sich durch Behandlung mit Sauerstoff enthaltenen Gasen z. B. Luft bei einer Temperatur von 350 bis 500°C und anschließende Reduktion regenerieren.

Bei der Spaltung der Formylvaleriansäureester in Stufe c hält man eine Temperatur von 50 bis 400°C vorzugsweise 60 bis 350°C insbesondere 100 bis 280°C, besonders vorteilhaft von 120 bis 200°C ein. Im allgemeinen wird die Spaltung unter Atmosphärendruck durchgeführt. Es ist jedoch auch möglich, verminderten Druck oder erhöhten Druck zweckmäßig im Bereich von 10 mbar bis 20 bar anzuwenden. Im allgemeinen hält man eine Katalysatorbelastung von 0,01 bis 40, vorzugsweise 0,1 bis 20 kg Formylvaleriansäureester je kg Katalysator und Stunde ein.

Es kann zweckmäßig sein, die Spaltung der Formylvaleriansäureester in Stufe c unter zusätzlicher Mitverwendung von Verdünnungsmitteln durchzuführen. Geeignete Verdünnungsmittel sind z.B. Wasser, Alkohole wie Methanol, Ethanol, Butanol oder Cyclohexanol ferner Ether wie Dioxan oder Tetrahydrofuran sowie chlorierte Kohlenwasserstoffe wie Methylenchlorid, Chloroform, 1,2-Dichlormethan oder weiterhin aliphatische, cycloaliphatische oder aromatische Kohlenwasserstoffe wie Benzol, Toluol, Cyclohexan, Paraffine, ferner Ester wie Essigsäureester oder Propionsäureester.

Vorteilhaft wird der Alkohol verwendet, der dem in den Formylvaleriansäureestern gebundenen Alkohol entspricht, Edukt und Produkt sind so durch eine ausreichende Siedepunktdifferenz destillativ gut trennbar. Es hat sich bewährt, wenn das Molverhältnis von Formylvariansäureestern zu Verdünnungsmitteln 1:0,1 bis 1:50 insbesondere 1:0,5 bis 1:20 beträgt. Besonders bevorzugte Verdünnungsmittel sind Wasser und Alkanole mit 1 bis 6 Kohlenstoffatomen, insbesondere Methanol.

Vorteilhaft führt man die Spaltung in Stufe c unter Mitverwendung von molekularem Sauerstoff oder molekularen Sauerstoff enthaltenden Gasen die neben Sauerstoff Inertgase wie Stickstoff, Kohlendioxid, Argon oder Wasserdampf enthalten durch. Vorzugsweise wendet man ein Molverhältnis von Formylvaleriansäureestern zu molekularem Sauerstoff von 1:0,05 bis 1:3, insbesondere 1:0,2, bis 1:1,5 z.B. 1:0,25 bis 1,25 an. Hierdurch wird die Lebensdauer des Katalysators erhöht und insbesondere die Ausbeute an Pentensäureestern gesteigert. Die Mitverwendung von molekularem Sauerstoff war nicht angezeigt, da 5-Formylvaleriansäuremethylester schon bei 50°C durch molekularen Sauerstoff mit 96 %iger Ausbeute zu Adipinsäuremonomethylester oxidiert wird, wie aus der europäischen Patenschrift 131 860 bekannt ist und somit zu erwarten war, daß 4- und 3-Formylvariansäureester analog zu 2-Methylglutarsäure und 3-Ethylbernsteinsäureester oxidiert werden.

Die Umsetzung in Stufe c kann diskontinuierlich oder kontinuierlich als Festbettreaktion mit Festbettkatalysatoren beispielsweise in Sumpf oder Rieselfahrweise in der Flüssig- oder Gasphase oder als Wirbelbettreaktion mit in auf- und abwirbelnder Bewegung befindlichen Katalysatoren in der Gasphase oder in der Flüssigphase mit löslichen Katalysatoren oder suspendierten Trägerkatalysatoren durchgeführt werden.

Eine bevorzugte Ausführungsform in der Stufe c in Flüssigphase sieht z. B. so aus, daß man Formylvaleriansäureester und gegebenenfalls Verdünnungsmittel mit Sauerstoff enthaltenen Gasen bei einer Temperatur unterhalb des Siedepunkts des Formylvaleriansäureesters über einen festen Katalysator leitet oder in Gegenwart eines suspendierten festen Katalysators oder eines gelösten Homogenkatalysators erhitzt. Das flüssige Reaktionsprodukt wird anschließend nach Abtrennung der Katalysatoren durch Destillation in Pentensäureester sowie gegebenenfalls Verdünnungsmittel und nicht umgesetzte Formylvariansäureester getrennt.

Eine bevorzugte Ausführungsform der Stufe C des erfindungsgemäßen Verfahrens in der Gasphase wird z. B. so durchgeführt, daß man ein Gemisch aus Formylvaleriansäureester und gegebenenfalls Verdünnungsmittel verdampft und dann zusammen mit Luft und zweckmäßig zusätzlich einem Trägergas wie Stickstoff, Kohlendioxid oder Argon bei der vorgenannten Temperatur gasförmig in eine fest angeordnete oder eine in auf- und abwirbelnder Bewegung befindliche Katalysatorschicht einleitet. Der Reaktionsaustrag wird kondensiert und anschließend durch fraktionierende Destillation aufgetrennt. Nicht umgesetzte Formylvaleriansäureester werden zweckmäßig in die Stufe c zurückgeführt. Das erhaltene Gemisch aus 4-, 3- und 2-Pentensäureestern wird wiederum in die Hydroformylierungsstufe a gegebenenfalls mit Pentensäureestern, die in Stufe b anfallen, zur Herstellung von 5-Formylvaleriansäureestern zurückgeführt. Die als Nebenprodukte anfallenden Valeriansäureester können ausgeschleust oder ebenfalls als Lösungsmittel in die Stufe a zurückgeführt werden.

Nach dem erfindungsgemäßen Verfahren ist es möglich, Pentensäureester durch Hydroformylierung mit hoher Ausbeute in 5-Formylvaleriansäureester zu überführen, da die als Nebenprodukte anfallenden isomeren Formylvaleriansäureester nach Spaltung in Pentensäureester wiederum in die Hydroformylierung eingehen.

5-Formylvaleriansäureester, die nach dem Verfahren der Erfindung erhältlich sind, eignen sich z. B. zur Herstellung von Aminocapronsäureester. Das Verfahren nach der Erfindung sei in folgenden Beispielen veranschaulicht.

Beispiel 1 bis 8

Stufe a

Die Hydroformylierungsversuche wurden in einer kontinuierlichen Arbeitsweise durchgeführt. Die Vorrichtung besaß eine Flüssigdosierpumpe, mit der Pentensäuremethylester und der gelöste Cobaltkatalysator in die zwei hintereinander geschaltete Rührautoklaven gefördert wurden. Das Synthesegas wurde druckgeregelt mit dem Flüssigzulauf vor dem ersten Reaktor zusammengeführt. Die beiden Reaktoren besaßen Flüssigvolumina von 1,2 bzw. 1,12 1. Der zweiphasige Austrag wurde unter Druck in einem Behälter gesammelt, aus dem eine bestimmte Abgasmenge abgeregelt wurde. Die Flüssigphase wurde standgeregelt in ein Vorlagegefäß entspannt. In den Beispielen 1 - 2 wurde $Co_2(CO)_8$ in der angegebenen Menge als Katalysator und ein Synthesegas mit $CO : H_2 = 1$ verwendet.

Die Beispiele 1 - 3 beschreiben den Einfluß der Cobaltkonzentration auf Umsatz, Linearanteil des Aldehydgemisches und Hochsiederbildung.

Die Beispiele 4, 5 beschreiben den Temperatureinfluß bei konstanter Cobaltkonzentration auf Umsatz, Linearanteil und Hochsiederbildung.

Die Beispiele 6 - 8 beschreiben den Einfluß des Drucks auf Umsatz, Linearanteil und Bildung von Hochsiedern.

Tabelle 1: Ergebnisse der Hydroformylierung von 3-Pentensäuremethylester (PSE)

Zulauf: 3-Pentensäuremethylester mit der entsprechenden Menge an gelöstem $Co_2(CO)_8$

Belastung 0,15 $l \times l^{-1} \times h^{-1}$

| Bsp. | $\dfrac{\text{Mol Co}}{\text{Mol PSE}} \times 100\ [\%]$ | Druck/bar | Temp./°C | % Umsatz | % n-Anteil[1] | % (m/m) Hochsieder im Austrag |
|---|---|---|---|---|---|---|
| 1 (Vergleich) | 2 | 200 | 110 | 97,7 | 55 | 52[2] |
| 2 | 0,48 | 200 | 110 | 86,6 | 59,6 | 37[2] |
| 3 | 0,16 | 200 | 110 | 69,7 | 68,0 | 14,6[2] |
| 4 | 0,16 | 200 | 100 | 37,2 | 69,5 | 3,5[2] |
| 5 | 0,16 | 200 | 90 | 17,6 | 69 | 0,3[3] |
| 6 | 0,16 | 280 | 100 | 39,5 | 69,1 | 8,8[2] |
| 7 | 0,16 | 130 | 100 | 33,2 | 69,4 | 1,7[3] |
| 8 | 0,16 | 90 | 100 | 32,8 | 68,7 | < 0,3[3] |

[1] Anteil an 5-Formylvaleriansäuremethylester am gesamten Formylvaleriansäuremethylester

[2] Bestimmung über GC (int. Standardmethode)

[3] Bestimmung über Destillation

[4] Summe aller isomeren Formylvaleriansäureester (FVSE)

Tabelle 1 Fort.: Ergebnisse der Hydroformylierung von 3-Pentensäuremethylester
Zulauf: 3-Pentensäuremethylester mit der entsprechenden Menge
an gelöstem $Co_2(CO)_8$
Belastung 0,15 $l \times l^{-1} \times h^{-1}$

| Bsp. | % Sel. Valerianester | % Sel. FVSE[4] |
|---|---|---|
| 1 (Vergleich) | 5,6 | 42,3 |
| 2 | 2,9 | 60,1 |
| 3 | 2,1 | 83,2 |
| 4 | 0,8 | 94,4 |
| 5 | 1,7 | 97,4 |
| 6 | 1,9 | 89,0 |
| 7 | 1,6 | 96,5 |
| 8 | 0,9 | 98,8 |

Beispiel 9

Stufe a

In der gleichen Apparatur wie in Beispiel 2 wurde 3-Pentensäuremethylester mit 0,16 Mol-% Cobalt mit einer Belastung von 0,3 anstelle von 0,15 $l \times l^{-1} \times h^{-1}$ bei 100°C und 200 bar $CO/H_2$ umgesetzt. Bei einem Umsatz von 15,5 % wurde eine Gesamtselektivität zu Formylvaleriansäureestern von 98,7 % mit einem Linearanteil von 70,5 % erzielt. Vom umgesetzten Pentenester wurden 1,2 % zu Valerianester hydriert.

Beispiel 10 (Variation der Synthesegaszusammensetzung)

Stufe a

Man verfährt analog Beispiel 7 jedoch mit einem Verhältnis CO : $H_2$ wie 6 : 4 statt 1 : 1. Es wurde ein Umsatz von 19,8 % und eine Gesamtselektivität zu Formylvalerianestern von 99,2 % bei einem Linearanteil von 68,9 % erzielt. Vom umgesetzten Pentenester wurden 0,8 % zu Valerianester hydriert.

Beispiel 11 (N-Basen-Einfluß auf Katalysatoraktivität)

Stufe a

Anstelle von $Co_2(CO)_8$ wird eine Katalysatorlösung aus einer vorangegegangenen Hydroveresterung von Butadien zu Pentensäuremethylester eingesetzt. Nach Abdestillation von Pentenester und leichter siedenden Komponenten wird ein Teil des Sumpfes als Katalysator für die Hydroformylierung eingesetzt. Dieser Sumpf enthält 2,2 Mol Pyridin, bezogen auf Cobalt. Bei 130 bar/100°C wird Pentensäuremethylester mit diesem Katalysator (0,16 Mol-% Co) analog Beispiel 7 hydroformyliert. Bei einem Umsatz von 33 % erhält man Formylvalerianmethylester mit einer Selektivität von 97 % und einem Linearanteil von 68 %; ca. 2 % des umgesetzten Pentenesters wird zu Valerianester hydriert.

Beispiel 12

Stufe a

Analog Beispiel 7 wurde eine Mischung von 3-Pentensäuremethylester mit 800 ppm Cobalt (als $CO_2$-(CO)$_8$ (360 ml/h) bei 100°C und 130 bar (CO/$H_2$ = 1) hydroformyliert. Es wurde ein Austrag von 327 g/h erhalten, der sich wie folgt zusammensetzt:
38,1 % (m/m) Formylvalerianester (mit 69,6 % n-Anteil)
0,4 % (m/m) Valeriansäuremethylester
61,2 % (m/m) Pentensäuremethylester
Dieses Ergebnis entspricht einem Umsatz von 33 %, einer Selektivität an gesamten Formylvalerianester von 98,8 % une einer Selektivität an 5-Formylvalerianester von 68,8 %.
Dieser Austrag wurde mit stündlich 150 ml 5 %iger Essigsäure unter Einleiten von 5 l/h Luft unter guter Durchmischung durch ein Rohr geleitet. Nach der Phasentrennung wurden 154 ml einer 0,2 %igen Cobaltacetatlösung (ber. als Cobalt) abgetrennt.
In einer diskontinuierlichen Destillation wurden 835 g der organischen Phase in ca. 3 g Valeriansäuremethylester, 495 g 3-Pentensäuremethylester, 12 g 2-trans-Pentensäuremethylester sowie ca. 315 g eines 5-, 4-, 3-Formylvaleriansäuremethylester-Gemisches und 6 g Rückstand getrennt.

Stufe b

Das Formylvaleriansäuremethylester-Gemisch wurde durch weitere fraktionierende Destillation in 215 g 5-Formylvaleriansäuremethylester (99 %ig), 90 g eines Gemisches aus 5-, 4- und 3-Formylvaleriansäuremethylester (2 % 5-, 70 % 4- und 28 % 3-FVSE) und 10 g Rückstand aufgetrennt.

Stufe c

Innerhalb einer Stude wurden 90 g des Gemisches aus 5-, 4- und 3-Formylvaleriansäuremethylester zusammen mit 180 g Methanol in einen Verdampfer gepumpt und von dort zusammen mit 70 l Luft bei 250°C über 200 g eines $SiO_2$-Trägerkatalysators geleitet, der jeweils 0,5 % Ru, Rh und Pt enthielt. Bei der destillativen Aufarbeitung der kondensierten Reaktionsausträge (260 g) wurden nach Abtrennung von Methanol und Wasser 15 % 4-, 53 % 3- und 15 % 2-trans-Pentensäuremethylester und 17 % Valeriansäuremethylester erhalten. Außerdem wurden 10 g eines Gemisches aus 4- und 3-Formylvaleriansäuremethylester zurückgewonnen.

EP 0 295 549 B1

Stufe d

Das Gemisch aus 4-, 3-, 2-Pentensäuremethylester wurde in Stufe a zurückgeführt und ließ sich auch in Gegenwart des Valeriansäuremethylesters mit praktisch gleicher Ausbeute wie ausgehend von 3-Pentensäuremethylester für die Hydroformylierung wiederverwenden.

Beispiel 13 - 15

Stufe c

Über 20 g eines Festbettkatalysators wurden 30 ml/h Formylvaleriansäuremethylester-Gemisch (35 % 3-FVSE und 57 % 4-FVSE), die nach Beispiel 8 und anschließender fraktionierender Destillation des Reaktionsaustrags erhalten worden waren, zusammen mit 30 l/h Trägergas der genannten Zusammensetzung geleitet. Die gasförmigen Reaktionsprodukte wurden kondensiert und gaschromatographisch analysiert.

| Nr. | Katalysator | N₂/Luft [l/h] | PSE [Gew.-%] | Umsatz [%] | Selektivität [%] |
|---|---|---|---|---|---|
| 13 | 0,75 % Pd + 0,1 % V auf SiO | 24/6 | 47,4 | 81 | 62 |
| 14 | 0,75 % Pd + 0,04 % Co auf SiO₂ | 18/12 | 69,7 | 87 | 84 |
| 15 | 0,75 % Pd + 0,06 % Mn auf SiO₂ | 18/12 | 74,7 | 91 | 83 |

**Patentansprüche**

1. Verfahren zur Herstellung von 5-Formylvaleriansäureestern welches folgende Schritte umfaßt
   a) Hydroformylierung von Pentensäureestern durch Umsetzen mit Kohlenmonoxid und Wasserstoff bei einer Temperatur von 60 bis 160 °C und erhöhtem Druck in Gegenwart von Carbonylkomplexen von Metallen der 8. Gruppe des periodischen Systems unter Bildung eines Gemisches von 5-, 4- und 3-Formylvaleriansäureestern
   b) Abtrennung von 5-Formylvaleriansäureestern aus dem so erhaltenen Gemisch von 5-, 4- und 3-Formylvaleriansäureestern unter Verbleib eines im wesentlichen aus 4- und 3-Formylvaleriansäureester bestehenden Gemisches
   c) Spalten des im wesentlichen aus 4- und 3-Formylvaleriansäureester bestehenden Gemisches unter Bildung von Pentensäureestern bei einer Temperatur von 50 bis 400 °C in Gegenwart von Katalysatoren, die ein oder mehrere Metalle der 8. Nebengruppe des Periodischen Systems enthalten, und
   d) Zurückführen der Pentensäureester in Stufe a zur Hydroformylierung.

2. Verfahren nach Anspruch 1, dadurch gekennzeichnet, daß man in Stufe a eine Kobaltkonzentration von 0,05 Mol.% bis 0,3 Mol.%, bezogen auf Pentensäureester einhält.

3. Verfahren nach den Ansprüchen 1 und 2, dadurch gekennzeichnet, daß man in der Stufe a einen Umsatz an Pentensäureestern von 10 bis 50 % einhält.

4. Verfahren nach den Ansprüchen 1 bis 3, dadurch gekennzeichnet, daß man in Stufe a) eine Temperatur von 80 bis 110 °C einhält.

5. Verfahren nach den Ansprüchen 1 bis 4, dadurch gekennzeichnet, daß man Trägerkatalysatoren verwendet, die mindestens zwei Edelmetalle der 8. Nebengruppe des Periodischen Systems enthalten.

6. Verfahren nach den Ansprüchen 1 bis 4, dadurch gekennzeichnet, daß man Trägerkatalysatoren verwendet, die mindestens ein Edelmetall der 8. Nebengruppe des Periodischen Systems und mindestens ein Metall ausgewählt aus der Gruppe Eisen, Kobalt und Nickel enthalten.

9

7. Verfahren nach den Ansprüchen 1 bis 6, dadurch gekennzeichnet, daß man in Stufe c einen Katalysator verwendet, der zusätzlich mindestens eines der Elemente Kupfer, Silber, Zink, Titan, Vanadium, Chrom, Molybdän, Wolfram, Mangan oder Rhenium enthält.

8. Verfahren nach den Ansprüchen 1 bis 7, dadurch gekennzeichnet, daß man die Umsetzung in Stufe c bei einer Temperatur von 60 bis 350°C durchführt.

9. Verfahren nach den Ansprüchen 1 bis 8, dadurch gekennzeichnet, daß man die Spaltung in Stufe c unter Zusatz von molekularem Sauerstoff oder solchen enthaltenen Gasen durchführt.

10. Verfahren nach den Ansprüchen 1 bis 9, dadurch gekennzeichnet, daß man ein Molverhältnis von Formylvaleriansäureester zu molekularem Sauerstoff von 1 : 0,05 bis 1 : 3 einhält.

11. Verfahren nach den Ansprüchen 1 bis 10, dadurch gekennzeichnet, daß man in Stufe c) Verdünnungsmittel mitverwendet.

12. Verfahren nach den Ansprüchen 1 bis 11, dadurch gekennzeichnet, daß man die Umsetzung in Stufe c unter Mitverwendung von Wasser oder Alkanolen mit 1 bis 6 Kohlenstoffen oder Gemischen derselben durchführt.

## Claims

1. A process for the preparation of a 5-formylvaleric ester which comprises the following steps:
   a) hydroformylation of a pentenoic ester by reaction with carbon monoxide and hydrogen at from 60 to 160°C and under superatmospheric pressure in the presence of a carbonyl complex of a metal of group 8 of the Periodic Table with formation of a mixture of 5-, 4-and 3-formylvaleric esters,
   b) isolation of the 5-formylvaleric ester from the resulting mixture of 5-, 4- and 3-formylvaleric esters, a mixture essentially consisting of 4- and 3-formylvaleric esters remaining,
   c) cleavage of the mixture essentially consisting of 4- and 3-formylvaleric esters at from 50 to 400°C in the presence of a catalyst, which contains one or more metals of subgroup 8 of the Periodic Table, to give pentenoic esters and
   d) recycling of the pentenoic esters to stage a for hydroformylation.

2. A process as claimed in claim 1, wherein, in stage a, a cobalt concentration of from 0.05 to 0.3 mol %, based on the pentenoic ester, is maintained.

3. A process as claimed in claim 1 or 2, wherein, in stage a, a conversion of pentenoate of from 10 to 50% is maintained.

4. A process as claimed in any of claims 1 to 3, wherein, in stage a, a temperature of from 80 to 110°C is maintained.

5. A process as claimed in any of claims 1 to 4, wherein the supported catalyst used contains two or more noble metals of subgroup 8 of the Periodic Table.

6. A process as claimed in any of claims 1 to 4, wherein the supported catalyst used contains one or more noble metal of subgroup 8 of the Periodic Table and one or metals selected from the group consisting of iron, cobalt and nickel.

7. A process as claimed in any of claims 1 to 6, wherein, in stage c, the catalyst used additionally contains one or more of the elements copper, silver, zinc, titanium, vanadium, chromium, molybdenum, tungsten, manganese and rhenium.

8. A process as claimed in any of claims 1 to 7, wherein the reaction in stage c is carried out at from 60 to 350°C.

9. A process as claimed in any of claims 1 to 8, wherein the cleavage in stage c is carried out with the addition of molecular oxygen or of a gas containing molecular oxygen.

**10.** A process as claimed in any of claims 1 to 9, wherein a molar ratio of formylvaleric ester to molecular oxygen of from 1 : 0.05 to 1 : 3 is maintained.

**11.** A process as claimed in any of claims 1 to 10, wherein a diluent is present in stage c).

**12.** A process as claimed in any of claims 1 to 11, wherein the reaction in stage c is carried out in the presence of water or an alkanol of 1 to 6 carbon atoms or a mixture of these.

**Revendications**

1. Procédé de préparation d'esters de l'acide 5-formylvalérique, comprenant les étapes suivantes:
   a) Hydroformylation d'esters d'acide penténique par réaction avec du monoxyde de carbone et de l'hydrogène à une température de 60 à 160°C et sous pression elevée, en présence de complexes de carbonyle et de métaux du groupe VIII du système périodique, avec formation d'un mélange d'esters d'acides 5-, 4- et 3-formylvalériques,
   b) Séparation des esters d'acide 5-formylvalérique d'avec le mélange ainsi obtenu d'esters d'acides 5-, 4- et 3-formylvalériques, ce qui laisse un mélange composé essentiellement d'esters d'acides 4- et 3-formylvalériques,
   c) Dissociation du mélange composé essentiellement d'esters d'acides 4- et 3-formylvalériques, avec formation d'esters d'acide penténique, à une température de 50 à 400°C en présence de catalyseurs qui contiennent un ou plusieurs métaux du groupe VIII du système périodique, et
   d) Renvoi des esters d'acide penténique dans l'étape a) pour leur hydroformylation

2. Procédé selon la revendication 1, caractérisé en ce qu'on maintient, dans l'étape a), une concentration de cobalt de 0,05 à 0,3% en moles, par rapport aux esters d'acide penténique.

3. Procédé selon la revendication 1 ou 2, caractérisé en ce qu'on maintient, dans l'étape a), un degré de transformation des esters d'acide penténique de 10 à 50%.

4. Procédé selon l'une quelconque des revendications 1 à 3, caractérisé en ce qu'on maintient, dans l'étape a), une température de 80 à 110°C.

5. Procédé selon l'une quelconque des revendications 1 à 4, caractérisé en ce qu'on utilise des catalyseurs sur support qui contiennent au moins deux métaux précieux du groupe VIII du système périodique.

6. Procédé selon l'une quelconque des revendications 1 a 4, caractérisé en ce qu'on utilise des catalyseurs sur support qui contiennent au mains un métal précieux du groupe VIII du système périodique et au moins un métal choisi dans le groupe du fer, du cobalt et du nickel.

7. Procédé selon l'une quelconque des revendications 1 à 6, caractérisé en ce qu'on utilise, dans l'étape c), un catalyseur qui contient en plus l'un au moins des éléments cuivre, argent, zinc, titane, vanadium, chrome, molybdène, tungstène, manganèse et rhénium.

8. Procédé selon l'une quelconque des revendications 1 à 7, caractérisé en ce qu'on conduit la réaction, dans l'étape c), à une température de 60 a 350°C.

9. Procédé selon l'une quelconque des revendications 1 à 8, caractérisé en ce qu'on effectue la dissociation, dans l'étape c), avec addition d'oxygène moléculaire ou de gaz contenant celui-ci.

10. Procédé selon l'une quelconque des revendications 1 à 9, caractérisé en ce qu'on maintient un rapport molaire des esters d'acides formylvalériques à l'oxygène moléculaire de 1 : 0,05 à 1 : 3.

11. Procédé selon l'une quelconque des revendications 1 à 10, caractérisé en ce qu'on utilise simultanément, dans l'étape c), un diluant.

12. Procédé selon l'une quelconque des revendications 1 à 11, caractérisé en ce que la réaction dans l'étape c) est conduite avec utilisation simultanée d'eau, d'alcanols à 1-6 atomes de carbone ou de mélanges de ceux-ci.